# EUROPEAN PATENT APPLICATION

(11) **EP 0 621 477 A2**
(43) Date of publication of application: **26.10.1994**
(21) Application number: 94104632.8
(22) Date of filing: 23.03.1994
(51) Int. Cl.: G01N 33/483, A61B 3/10, A61B 19/00, A61F 9/00, G06K 9/00

(54) **Method for obtaining a quantitative representation of the cell morphology of a biological tissue**

(30) Priority: 23.03.1993 JP 89244/93
(71) Applicant: KONAN INC., Nishinomiya-shi, Hyogo (JP)
(72) Inventor: Takahashi,Yohko, c/o Konan Inc., Nishinomiya-shi, Hyogo (JP); Kasahara, Tatsuya, c/o Konan Inc., Nishinomiya-shi, Hyogo (JP)
(74) Representative: Glawe, Delfs, Moll & Partner

(57) **Abstract**

In examining the progress of patients before and after a cataract operation, all data can be computed upon the morphology of cornea endothelium cells for use of medical treatment, from a cornea endothelium cell image with less labor.

Positional data of center points of two-dimensionally continuous cells of a cornea endothelium cell image are entered into a computer. Center points of the cells within a specified distance from a given center point are sorted clockwise (101-104). If an angle formed by two angularly successive points and the center point is less than a specified angle, the point having the greater distance from the center point is excluded (105-113). If the distance between the first point and the third point of three angularly successive points is shorter than the distance between the given center point and the second point, the second point is excluded (114-122). In this way, for each given cell a set of peripheral points which are center points of cells directly adjacent to the given cells is obtained. The average distances between the peripheral points and the center point of each given cell are determined (123). A perpendicular line that proportionally divides the distance between a center point and any of its peripheral points proportional to the ratio of their average distances to the peripheral points is determined, and intersection points of the resulting perpendicular lines are determined as vertices of the polygonal outline of the cells (124-131). Thus, morphological data are computed from the obtained vertices (132).

## Description

The invention relates to a method for obtaining a quantitative representation of the cell morphology of a biological tissue, and more specifically a method for quantitatively computing morphology of cornea endothelium cells to provide decision data to doctors, in examining the progress of patients before and after a cataract operation in the ophthalmic field of medical treatment.

### [Prior Art]

As the aging society progresses, the number of cataract patients has been increasing. It is common practice, almost mandatorily, that cornea endothelium cells are checked up before and after a cornea operation. As the method of the checkup, there has conventionally been implemented specular photography (photography of cornea endothelium cells by reflected rays of light that has been projected on the cornea), in which the following methods are available to obtain decision reference data:

### 1. Grid method:

With a grid superimposed on a magnified photograph of cornea endothelium or the like, the number of cells in the grid of specified intervals is counted and converted into the number of cells per mm² (cell density).

### 2. Cell sizer method:

A model whose cell density and coefficient of variation (CV) have been known is previously prepared, and the cell density and the CV are determined by comparing it with a cornea endothelium photograph or the like.

### 3. Digitizer method:

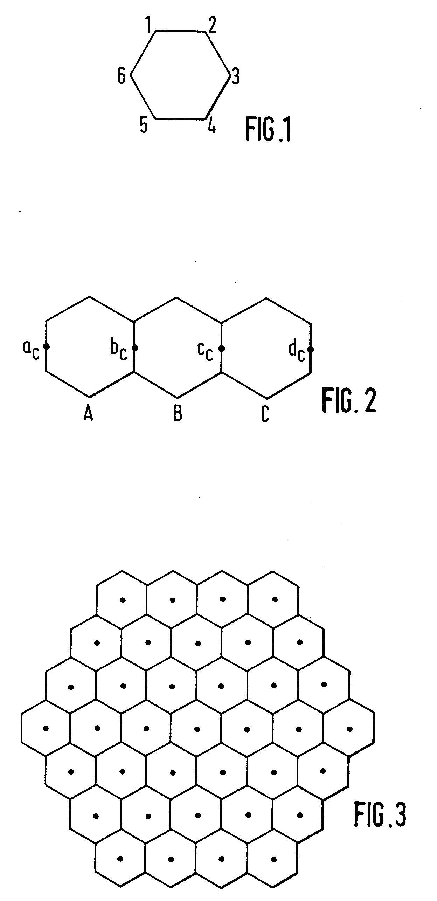

Coordinates of vertices of a cell (e.g. coordinates of points 1, 2, 3, 4, 5, 6, etc. which are vertices of the profile of a hexagonal cell as show in FIG. 1) are entered into a computer or the like, and the correct morphology of the individual cells is determined by the following equations:

$\text{Equation 1:} \text{Cell area} \text{:} \text{Ar} \text{=} \frac{\text{1}}{\text{2}} \text{Σ {(} {\text{X}}_{\text{n}} \text{×} {\text{Y}}_{\text{n+}} {\text{}}_{\text{1}} \text{)-(} {\text{X}}_{\text{n+}} {\text{}}_{\text{1}} \text{×} {\text{Y}}_{\text{n}} \text{)} (µ} \text{m} \text{²)}$ 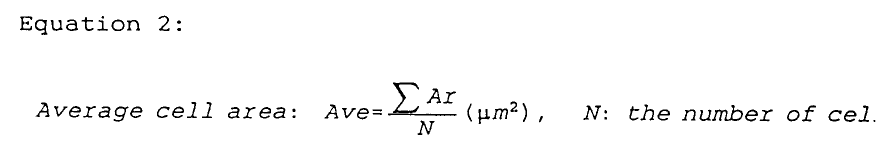
$\text{Equation 3:} \text{Cell density} \text{:} \text{CD} \text{=1000000/} \text{Ave} \text{(} \text{pcs} \text{/} \text{m} \text{²)}$ 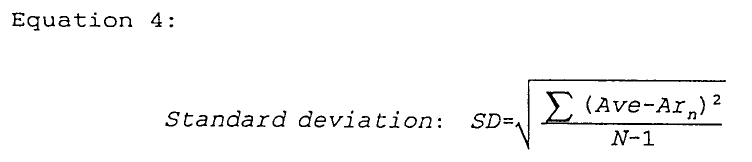
$\text{Equation 5:} \text{Coefficient of variation} \text{:} \text{CV} \text{=} \text{SD} \text{/} \text{Ave}$

$\text{Equation 6:} \text{Hexagonal cell frequency} \text{:} \text{Ap} \text{₆=(} \text{number of hexagonal cells} \text{)/N}$

### 4. Opposite-side input method:

With inputs of midpoints *a*_{c}, *b*_{c}, *c*_{c}, ... of opposite two sides of mutually adjoining cells A, B, C, ... (see FIG. 2), the cell area is approximated by determining the area of an equilateral hexagon circumscribing a circle whose diameter is formed by each two points of the midpoints. In this case, for example, the area of the cell A is as follows: 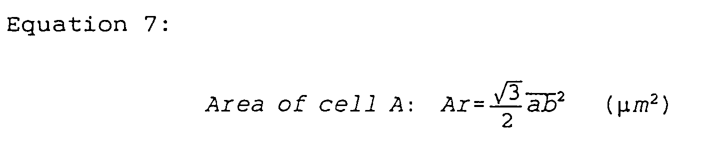
The above-described conventional methods have the following problems:
1. The grid method allows only the determination of cell density;
2. The cell sizer method, although allowing determination of cell density and coefficient of variation, would result in variation among persons who execute the comparison and have difficulties in obtaining correct values;
3. The digitizer method, although allowing all morphological data needed for clinical treatment to be obtained, would necessitate a great deal of input labor, thus unsuitable for practical use; and
4. The opposite-side input method, although very simple in input, could not afford to obtain the polygonal number (hexagonal cell frequency, which is a decision criterion for doctors).

The present invention has been developed in view of the above-mentioned problems. An object of the present invention is therefore to provide a method for computing morphology of cornea endothelium cells, which solves the various disadvantages of the conventionally practiced methods and which allows profiles of cells to be reproduced simply by entering center points of the cells of a two-dimensionally continuous cell image, so that all morphological data needed for clinical treatment equivalent to the digitizer method can be obtained with input labor far less than in the digitizer method.

For achieving the object the invention provides a method as defined in claim 1.

In a preferred embodiment, the
invention provides a method for computing morphology of cornea endothelium cells, which comprises, for every center point of individual cells of a two-dimensionally continuous cornea endothelium cell image such as a cornea endothelium cell photograph, the cell centers have been entered into a computer or the like: a step for determining peripheral points which are cell centers within a specified distance from a center point; a step for sorting clockwise or counterclockwise the peripheral points obtained by the preceding step; a step for, when an angle formed by successive two peripheral points obtained by the preceding step and the center point is less than a specified angle, excluding from the peripheral points either of the two peripherals points, whichever longer in distance from the center point; a step for, when a distance between the first point and the third point of successive three peripheral points obtained by the preceding step is shorter than a distance between the center point and the second point, excluding the second point from the peripheral points; a step for determining an average distance between the peripheral points obtained by the preceding step and the center point; a step for, after effecting all the preceding steps respectively on a plurality of two-dimensionally successive center points of cornea endothelium cells, determining a perpendicular line which proportionally divides a distance between the center point and a peripheral point by their average distances to the peripheral points, i.e. a line perpendicular to a line that passes the proportionally divided point to connect the center point and the peripheral point with each other, respectively for successive peripheral points, and determining intersection points of the resulting perpendicular lines as vertices of a profile of a cell; and a step for computing morphological data of cornea endothelium cells from coordinates of the vertices obtained by the preceding step, wherein the above steps are performed sequentially to quantitatively determine the morphology of the cornea endothelium cells.

In the step for determining peripheral points within a specified distance from a center point, it is advisable that the specified distance is a distance in a range of 2.5 to 3.5 times a distance from the center point to its shortest peripheral point.

In the step for, when an angle formed by successive two peripheral points and the center point is less than a specified angle, excluding from the peripheral points either of the two peripheral points, whichever longer in distance from the center point, it is advantageous that the specified angle is a value in a range of 20 to 40 degrees.

Also, in the step for, when an angle formed by successive two peripheral points and the center point is less than a specified angle, excluding from the peripheral points either of the two peripheral points, whichever longer in distance from the center point, it is desirable that the step further includes a step for, when the angle formed by successive two peripheral points and the center point is greater than a value of 90 to 110 degrees, the center point is taken as a center point of a peripheral cornea endothelium cell.

Further, in the step for, when a distance between the first point and the third point of successive three peripheral points is shorter than a distance between the center point and the second point, excluding the second point from the peripheral points, it is effective that the step further includes a step for, when the angle formed by the first point, the center point, and the third point is greater than a value of 120 to 140 degrees, suppressing the second point from being excluded from the peripheral points.

Yet further, in the step for determining a perpendicular line which proportionally divides a distance between the center point and a peripheral point by their average distances to the peripheral points, respectively for successive peripheral points, and determining intersection points of the resulting perpendicular lines as vertices of a profile of a cell, it is effective that the step further includes a step for weighting proportional allotment with values of the square to fourth power of the average distances and effecting the proportional division with the values.

In the method for computing morphology of cornea endothelium cells according to the present invention, for each of center points of a two-dimensionally continuous cell image such as a cornea endothelium cell photograph, the center points having been entered into a computer or the like, peripheral points which are cell centers within a specified distance from one center point are determined to necessarily include actual peripheral cells, and the resulting peripheral points (including peripheral points on the outer periphery of actual peripheral points) are sorted clockwise or counterclockwise, so that the peripheral points are tested in turn, whereby the subsequent steps can be carried out smoothly. Next, through a step for, when the angle formed by successive two peripheral points obtained by the preceding step and the center point is less than a specified angle, excluding from the peripheral points either of the two peripheral points, whichever longer in distance from the center point, and through a step for, when a distance between the first point and the third point of successive three peripheral points obtained by the preceding step is shorter than a distance between the second point and the center point, excluding the second point from the peripheral points, actual peripheral points are obtained, so that a cell-like configuration can be determined correctly whereas the cell is originally an equilateral hexagon and deformed into triangular to ten-odd polygons. Then, average distances between the peripheral points obtained by the preceding step and the center point, and besides a perpendicular line which proportionally divides a distance between the center point and a peripheral point by their average distances to the peripheral points is determined, respectively for the successive peripheral points. By further determining intersection points of the resulting perpendicular lines, vertices of the cell profile can be obtained. From the coordinates of the vertices obtained in this way, quantitative morphological data on the cornea endothelium cells can be computed.

Of the above steps, in the step for determining peripheral points within a specified distance from a center point, the specified distance is set to a distance between 2.5 to 3.5 times the distance from the center point to its shortest peripheral point. This is the shortest distance that allows actual peripheral cells to be necessarily included, and to be efficiently tested. In the step for, when the angle formed by successive two peripheral points and the center point is less than a specified angle, excluding either of the two peripheral points, whichever longer in distance from the center point, the specified angle is set to a value of 20 to 40 degrees. Further, in the step for, when the angle formed by successive two peripheral points and the center point is less than a specified angle, excluding from the peripheral points either of the two peripheral points, whichever longer in distance from the center point, the step further includes a step for, when the angle formed by the successive two peripheral points and the center point is greater than a value of 90 to 110 degrees, the center point is taken as a peripheral cell, so that the cell is suppressed from being reproduced. Still further, in the step for, when a distance between the first point and the third point of successive three peripheral points is shorter than a distance between the center point and the second point, excluding the second point from the peripheral points, the step further includes a step for, when the angle formed by the first point, the center point, and the third point is greater than a value of 120 to 140 degrees, suppressing the second point from being excluded from the peripheral points. These steps are well matched to actual cases, experimentally, making it possible to effectively determine actual peripheral points. Furthermore, in the step for determining a perpendicular line which proportionally divides a distance between the center point and a peripheral point by their average distances to the peripheral points, respectively for the successive peripheral points, to thereby determine intersection points of the resulting perpendicular lines as vertices of profiles of the cells, the step further includes a step for weighting proportional allotment with values of the square to fourth power of the average distances and effecting the proportional division with the resulting value. This step is also well matched to actual cases, experimentally, having a large effect.

Embodiments of the present invention are now described with reference to the accompanying drawings.
FIG. 1 is an explanatory view in entering vertices of the profile of a cell by the digitizer method;
FIG. 2 is an explanatory view in entering midpoints of two opposite sides of a cell by the opposite-side input method;
FIG. 3 is a schematic view in entering center points of cells of a two-dimensionally continuous cornea endothelium cell image;
FIG. 4 is a system diagram for entering center points of the cells;
FIG. 5 is another system diagram for entering center points of the cells;
FIG. 6 is a flow chart showing the steps (routine) performed by the computer;
FIG. 7 is a flow chart of the steps performed by the computer subsequent to FIG. 6;
FIG. 8 is a flow chart of the steps performed by the computer subsequent to FIG. 7; and
Fig. 9 is an explanatory view in determining the profiles of cells by entering the center points of the cells.

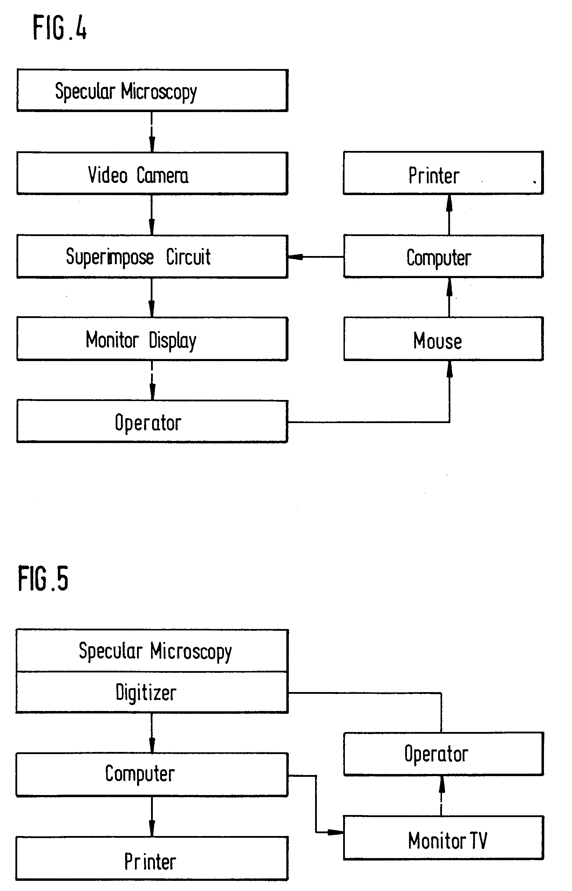

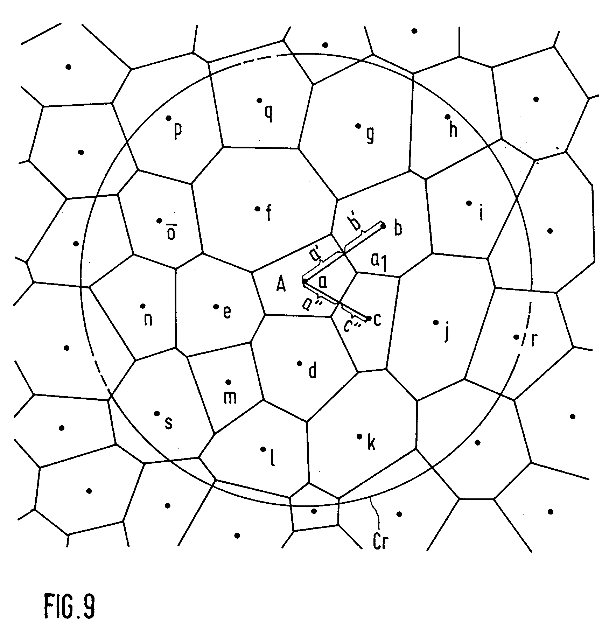

FIG. 3 shows a schematic diagram of a cell image which is applied when center points of cells of a two-dimensionally continuous cornea endothelium cell image such as a cornea endothelium cell photograph are entered into a computer. The cell is originally an equilateral hexagon and deformed into triangular to ten-odd polygons (see FIG. 9). In FIG. 3, however, the cell image is assumed to be a group of equilateral hexagonal cells and input points of the cells are represented by black points.

FIG. 4 is an embodiment of the system diagram applied to entry of the center points of the cells. In this case, a specular microscopy which is a cornea endothelium cell photography is photographed by a video camera. A video signal of a cornea endothelium cell image from the video camera is fed to a superimpose circuit, and the cell image is displayed on a monitor display. On the other hand, when the operator moves a mouse, the resulting movement signal is fed to the computer, and a movement signal of a cross mark due to the movement of the mouse is fed to the superimpose circuit from the computer so as to be superimposed on the image signal of cornea endothelium cells derived from the video camera. Accordingly, the operator can move the cross mark onto the cornea endothelium cell image on the monitor display, by moving the mouse. With the center of the cross mark positioned at a center (a black point as described above) of the cell image, the operator enters coordinates of the center of a cell by pressing the button of the mouse. This input operation is effected for every cell center (200 times for 200 cells). With cell centers entered in this way, the computer determines vertices of the profile of each cell according to the program loaded, and performs the computations as shown in the foregoing Equations 1 through 6 from the obtained coordinates of vertices to compute quantitative morphological data on the cells. The computer then prints out the obtained data by a printer.

FIG. 5 is another embodiment of the system diagram which is applied to entry of center points of the cells. In this case, a specular microscopy, which is a cornea endothelium cell photography, is placed on the upper plate surface of a digitizer. The operator moves the cursor of the digitizer, so that the center of a cross mark sited at the transparent portion of the cursor is positioned at the center point of each cell image. In this state, the operator presses the button for the cursor to enter coordinates of the center point into a computer. In doing this, instructions to the operator are displayed on a monitor television connected to the computer, allowing the operator to manipulate the computer in compliance with the instructions. With center points of the cells entered in this way, the computer operates according to the loaded program as in the foregoing embodiment to compute the aforementioned quantitative morphological data of the cells, printing out the data by a printer.

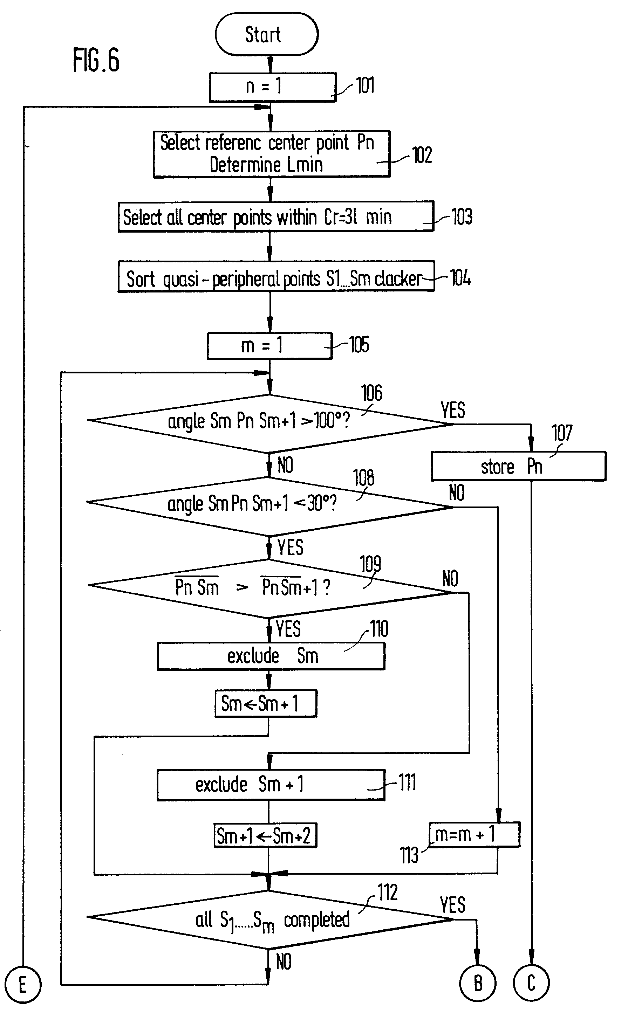

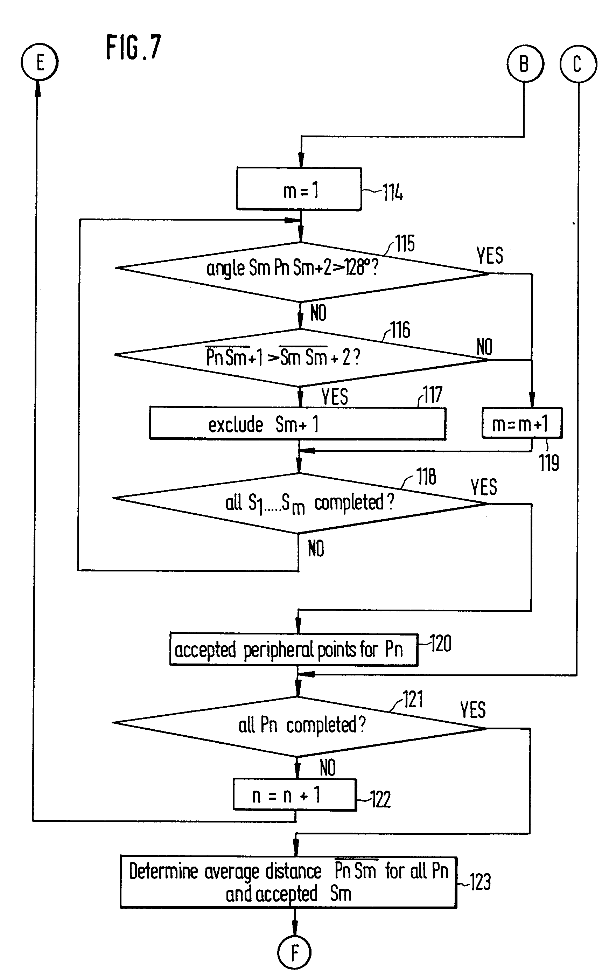

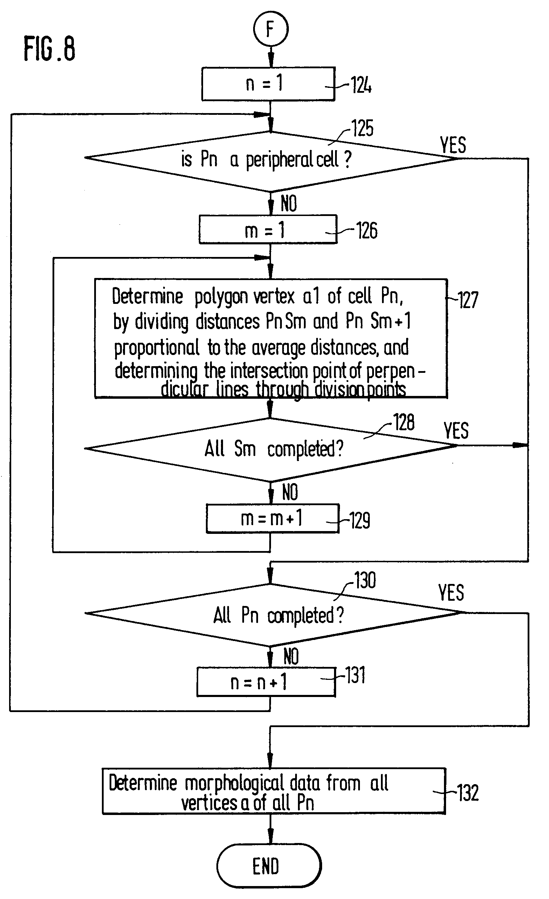

The steps (routine) of the program for computing morphological data of cells with center points of the cells of a cornea endothelium cell image entered into a computer are now described with reference to flow charts (FIGs. 6 to 8) and a cell input diagram (FIG. 9).

At the start, an arbitrary cell A is selected as the first one (n = 1) (step 101), and a distance Lₘᵢₙ = distance ac from point *a* as the center point Pₙ for n = 1 to its nearest center point c is calculated (step 102). Next, center points *b, c, d, e, f, g, h, i, j, k, l, m, n, o, p, q, r,* and *s* within a circle Cᵣ whose radius is three times Lₘᵢₙ, i.e. 3 × distance *ac*, from the center point *a* are selected and reserved as quasi-peripheral points Sₘ (step 103). Then the quasi-reference points obtained are sorted clockwise (according to the angle), so that these peripheral points can be tested in the order of *g, h, b, i, r, j, c, k, d, l, m, s, e, n, o, p, f*, and *q* (step 104). Subsequently, a peripheral point of m = 1, which is the first point of the successive quasi-peripheral points obtained by the preceding step, is selected (step 105), and it is checked whether or not the angle formed by g, which is the first quasi-peripheral point Sₘ (m = 1), a, which is the center point Pₙ, and h, which is the second quasi-peripheral point Sₘ₊₁ (m = 1), i.e. ∠gah is greater than 100 degrees (step 106). If it was greater than 100 degrees, the point Pₙ is stored as a center point of a second-type peripheral cell (step 107). However, when the angle ∠gah is not greater than 100 degrees, it is further checked whether or not the angle is less than 30 degrees (step 108). If it is not less than 30 degrees, the two peripheral points will be left as quasi-peripheral points and the value of m is raised by 1 (step 113), under which it is checked whether or not the tests for all the quasi-peripheral points have been completed (step 112), and the processing moves to a test for h, which is the new first quasi-peripheral point Sₘ (m = 2). Since, in this case the angle ∠gah is less than 30 degrees, a comparison is made between the two distances, i.e. the distances ag (PₙSₘ) and ah (PₙSₘ₊₁), which are distances from the center point a to the respective quasi-peripheral points g and h (step 109). If the distance PₙSₘ is longer than the distance PₙSₘ₊₁,Sₘ (m-1) will be excluded from the quasi-peripheral points, in which case all the remaining quasi-peripheral points following Sₘ are shifted up each by one, for example, Sₘ₊₁ to Sₘ, Sₘ₊₂ to Sₘ₊₁, Sₘ₊₃ to Sₘ₊₂, ..., so as to compensate the excluded Sₘ (step 110) and then, it is checked whether or not the tests for all the quasi-peripheral points have been completed (step 112), and thereafter the processing moves to a test for h (Sₘ), which is the new first quasi-peripheral point. Since, in this case, the distance ah is longer than the distance ag, the point h (Sₘ₊₁) is excluded, g (Sₘ) is left as a quasi-peripheral point and all the remaining quasi-peripheral points following Sₘ₊₁ are shifted up each by one, for example, Sₘ₊₂ to Sₘ₊₁, Sₘ₊₃ to Sₘ₊₂, Sₘ₊₄ to Sₘ₊₃, ...., so as to compensate the excluded Sₘ₊₁ (step 111), and then, it is checked whether or not the tests for all the quasi-peripheral points have been completed (step 112). Next, since the angle formed by g, which is the first quasi-peripheral point Sₘ (m = 1), a, which is the center point Pₙ, and b, which is the new second quasi-peripheral point Sₘ₊₁ (m = 1), i.e. the angle ∠gab is greater than 30 degrees (step 108), b is left as a quasi-peripheral point, and the value of m is increased by 1 (step 113), under which it is checked whether or not the tests for all the quasi-peripheral points have been completed (step 112), and thereafter the processing moves to a test for b, which is now the first quasi-peripheral point Sₘ (m = 2). Since the angle formed by b, which is the first quasi-peripheral point Sₘ (m = 2), a, which is the center point Pₙ, and i, which is the second quasi-peripheral point Sₘ₊₁ (m = 2), i.e. the angle ∠bai is less than 30 degrees (step 108) and further the distance ai is longer than distance ab (step 109), i is excluded and b is left as a quasi-peripheral point and all the remaining quasi-peripheral points following Sₘ₊₁ are shifted up each by one, for example, Sₘ₊₂ to Sₘ₊₁, Sₘ₊₃ to Sₘ₊₂, Sₘ₊₄ to Sₘ₊₃, ..., so as to compensate the excluded Sₘ₊₁ (step 111), and then, it is checked whether or not the tests for all the quasi-peripheral points have been completed (step 112). Next, since the angle formed by b, which is the first quasi-peripheral point Sₘ (m = 2), a, which is the center point Pₙ, and r, which is the second quasi-peripheral point Sₘ₊₁ (m = 2), i.e. the angle ∠bar is greater than 30 degrees (step 108), r is left as a quasi-peripheral point, and since the angle ∠raj is less than 30 degrees (step 108) and the distance ar is longer than distance aj (step 109), r is excluded and j is left as a quasi-peripheral point. In this way the sorted quasi-peripheral points will be checked one after another and the above steps are repeated until all the quasi-peripheral points are completely checked (from step 112 to B). As a result, the points, g, b, c, d, m, e, o and f are stored as the remaining quasi-peripheral points.

Of the quasi-peripheral points thus obtained, with respect to successive three quasi-peripheral points, a quasi-peripheral point of m = 1, which is the first point, is selected (step 114), and a comparison is made to check whether or not an angle ∠SₘPₙSₘ₊₂ formed by the first quasi-peripheral point Sₘ, the third quasi-peripheral point Sₘ₊₂, and the center point Pₙ is greater than 128 degrees (step 115). In this comparison, if the angle is greater than 128 degrees, the second quasi-peripheral point Sₘ₊₁ is not excluded from the stored peripheral points and the value of m is increased by 1 (step 119). Conversely, if the angle is not greater than 128 degrees, a comparison is made to check whether or not a distance SₘSₘ₊₂ between the first quasi-peripheral point Sₘ and the third quasi-peripheral point Sₘ₊₂ is shorter than a distance PₙSₘ₊₁ between the center point Pₙ and the second quasi-peripheral point Sₘ₊₁ (step 116). If the distance SₘSₘ₊₂ is shorter than the distance PₙSₘ₊₁, the second quasi-peripheral point Sₘ₊₁ is excluded from the stored peripheral points and all the remaining quasi-peripheral points following Sₘ₊₁ are shifted up each by one, for example, Sₘ₊₂ to Sₘ₊₁, Sₘ₊₃ to Sₘ₊₂, Sₘ₊₄ to Sₘ₊₃, ....., so as to compensate the excluded Sₘ₊₁ (step 117). In the case of the aforementioned successive three quasi-peripheral points g (Sₘ), b (Sₘ₊₁), and c (Sₘ₊₂), since the angle ∠gac is less than 128 degrees (step 115), the distance gc and distance ab are compared with each other (step 116), where distance ab is shorter than the distance gc, so that b is left as an actual peripheral point and the value of m is raised by 1 (step 119). Next, since the angle ∠bad is greater than 128 degrees, c is left as an actual peripheral point and the value of m is increased by 1. The next angle ∠cam is less than 128 degrees but the distance ad is shorter than the distance cm, and therefore d is left as an actual peripheral point. The angle ∠dae is less than 128 degrees, however, the distance am is longer than the distance de, and in this case, m is excluded from the peripheral points. In this way, the above described steps (routine) are repeated until all actual peripheral points are obtained (step 118). As a result, in this case, where Pₙ is the center point, a, of Cell A, peripheral points b, c, d, e and f are obtained and said peripheral points are stored (step 120). In the same manner, the peripheral points, which include the second-type peripheral points of the peripheral cells obtained by step 107, are stored for each center point of all the cells, continuously extending in a two dimensional pattern (step 121).

After step 121 is completed the average distances between the center points and the peripheral points are calculated and stored for all the center points of the cells, except of the second-type peripheral cells (step 123). The average distance between the center point and the peripheral points for Cell A, for example, is the sum of the distances from the center point, a, to the peripheral points b, c, d, e and f divided by the number of the peripheral points. Then, starting with a cell (n = 1) (step 124), it is checked whether or not its center point Pₙ is a second-type peripheral point of a peripheral cell (step 125). When Pₙ is not a second-type peripheral point, starting with the first peripheral point Sₘ (m = 1) (step 126) of Pₙ, a point of intersection of the first perpendicular line, which perpendicularly intersects the line PₙSₘ at the first dividing point divided with the ratio of the average distance about the point Pₙ obtained by step 123 and the average distance about the point Sₘ and the second perpendicular line, which perpendicularly intersects the line PₙSₘ₊₁ at the second dividing point divided with the ratio of the average distance about Pₙ and the average distance about Sₘ₊₁ is calculated, and then said intersecting point of the first perpendicular line and the second perpendicular line is stored as as a vertex of the cell whose center point is Pₙ (step 127). In this case, if Pₙ is the center point, a, of the cell A, the intersection point a₁ of the first perpendicular line to the line ab, passing through the first dividing point on the line ab, such that the line ab is divided with the proportionality of the average distance from the center point a to its peripheral points and the average distance from the center point b to its peripheral points, i.e. dividing at a ratio of a': b', and the second perpendicular line to the line ac, passing through the second dividing point on the line ac, such that the line ac is divided with the proportionality of the average distance from the center point a to its peripheral points and the average distance from the center point c to its peripheral points, i.e. dividing at a ratio of a'' : c'', is calculated and stored as one vertex of the cell A. In more detail, a': b' = average distance from the center point a to its peripheral points : average distance from the center point b to its peripheral points, and a'' : c '' = average distance from a to its peripheral points : average distance from c to its peripheral points. It has been found experimentally that the proportional division, if valued with the second to the fourth power of the average distances is applied (e.g. if the ratio of the average distances is 5 : 6, then the ratio of squaring is 25 : 36), can be well fitted to actual cases. The steps for determining all the vertices of Cell A are executed about all the peripheral points of the center point a (step 128 and step 129) and a pentagonal cell profile of Cell A, whose center point is a, can be obtained.

The steps for determining the form of a cell by determining the vertices of the profile of the cell are effected for all the center points of the two-dimensionally continuous cells (step 130 and step 131). Thus, from the vertices obtained for all the center points with peripheral points excluded, the whole morphological data such as average cell area, cell density, and hexagonal cell frequency are calculated by the above-mentioned equations of Equations 1 through 6.

In the above embodiments, morphological data on cornea endothelium cells have been computed by making analysis on a specular microscopy, which is a cornea endothelium cell photograph, with a video camera or a digitizer. However, it is also possible that, with a video camera loaded directly on a specular microscope, which is an eyeball microscope, an image signal from the camera is stored in a frame memory and analyzed to compute the aforementioned morphological data. The arrangement may be modified in various ways without departing the scope of the spirit of the present invention.

According to the method for computing morphology of cornea endothelium cells as described in Claim 1, in quantitatively computing morphology of cornea endothelium cells in the process of cornea endothelium cell checkup which is effected before and after a cataract operation, all the disadvantages that the conventional computation methods have had can be solved, and all morphological data necessary for clinical treatment can be easily obtained with far less labor than in the digitizer method that allows particularly accurate decision data to be obtained, simply by entering center points of the cells into a computer to reproduce the profiles of the cells from a cornea endothelium cell image such as of a specular microscopy.

According to the present invention as described in Claim 2, peripheral points, which are cell centers on the periphery of center points of the cells entered into the computer, can be determined efficiently without omissions. Thus, the time for computation can be reduced in computing the morphology of the cells.

According to the present invention as described in Claim 3, exclusion of unnecessary peripheral points from a large number of peripheral points including actual peripheral points on the periphery of a center point of one cell can be accomplished in a fashion well matched to actual cases.

According to the present invention as described in Claim 4, in excluding unnecessary peripheral points from a large number of peripheral points on the periphery of the center point of the one cell, if the center point of the one cell is the center point of a peripheral cell, the unnecessary peripheral points can be selected in a fashion well matched to actual cases. Therefore, the work of exclusion for the center point may be saved, so that exclusion of unnecessary peripheral points can be accomplished efficiently.

According to the present invention as described in Claim 5, in determining actual peripheral points by excluding unnecessary peripheral points from successive three peripheral points on the periphery of a center point of one cell, actual peripheral points can be determined easily and efficiently in a fashion well matched to actual cases.

According to the present invention as described in Claim 6, in determining a perpendicular line that proportionally divides a distance between a center point of one cell and a peripheral point thereof by their average distances to the peripheral points, for each of successive peripheral points, and then determining an intersection point of the perpendicular lines as a vertex of the profile of the cell, vertices that are well matched to actual cases can be determined so that the morphology of the cell can be determined.

## Claims

1. A method for obtaining a quantitative representation of the cell morphology of biological tissue, especially of cornea endothelium cells, comprising the steps of
a) obtaining a two-dimensional image of a selected area of the cell tissue;
b) determining the positions of the center points of cells in the image and entering the position data in a computer;
c) for each given cell (Pₙ) having a center point (a), selecting the center points (b, c, d, e, f) of peripheral cells (Sₘ) which are directly adjacent to the given cell (Pₙ);
d) for each given cell (Pₙ), determining the connecting lines from its center point (a) to the center points (b, c, d, e, f) of the adjacent peripheral cells (Sₘ) and constructing perpendicular lines on each connecting line at an intermediate point thereof;
e) determining the intersection point (a₁) of each two perpendicular lines;
f) determining a polygonal outline of the given cell (Pₙ) as determined by the intersection points (a₁) as polygon vertices;
g) repeating the foregoing steps for determining a polygonal outline for each cell.

2. The method as claimed in claim 1 , wherein the step of selecting the peripheral cells (Sₘ) adjacent to a given cell (Pₙ) (step c) comprises the steps of:
- selecting the center points which are within a specified distance from the center point of the given cell (Pₙ) as being quasi- peripheral points;
- sorting the thus determined quasi-peripheral point in clockwise or counterclockwise order with respect to the given center point (Pₙ);
- of two angularly successive quasi-peripheral points (Sₘ, Sₘ+1) being angularly spaced by less than a predetermined angle, selecting the point nearer to the given center point (Pₙ) as a peripheral point and excluding the other quasi-peripheral point having the greater distance from the given center point (Pₙ).

3. A method as claimed in claims 1 or 2 , wherein the intermediate points in step d) are determined by the steps of
h) for each given cell (Pₙ), determining the average distance from its center point (a) to the center points of the selected peripheral cells (Sₘ);
i) dividing each distance (Pₙ, Sₘ) between the center point (a) of a given cell (Pₙ) and the center points (b, c, d, e, f) of each neighbouring peripheral cell (Sₘ, Sₘ+1 ...) in proportion to the ratio of the average distances associated with the respective two cells or to a power of said ratio of average distances.

4. A method for quantitatively computing morphology of cornea endothelium cells from a cornea endothelium cell image, comprising, for every center point of individual cells of a two-dimensionally continuous cornea endothelium cell image:
a step for determining peripheral points which are cell centers within a specified distance from a center point;
a step for sorting clockwise or counterclockwise the peripheral points obtained by the preceding step;
a step for, when an angle formed by successive two peripheral points obtained by the preceding step and the center point is less than a specified angle, excluding from the peripheral points either of the two peripherals points, whichever longer in distance from the center point;
a step for, when a distance between the first point and the third point of successive three peripheral points obtained by the preceding step is shorter than a distance between the center point and the second point, excluding the second point from the peripheral points;
a step for determining an average distance between the peripheral points obtained by the preceding step and the center point;
a step for, after effecting all the preceding steps respectively on a plurality of two-dimensionally successive center points of cornea endothelium cells, determining a perpendicular line which proportionally divides a distance between the center point and a peripheral point by their average distances to the peripheral points, respectively for successive peripheral points, and determining intersection points of the resulting perpendicular lines as vertices of a profile of a cell; and
a step for computing morphological data of cornea endothelium cells from coordinates of the vertices obtained by the preceding step.

5. The method for computing morphology of cornea endothelium cells according to Claim 4, wherein in the step for determining peripheral points within a specified distance from a center point,
the specified distance is a distance in a range of 2.5 to 3.5 times a distance from the center point to its shortest peripheral point.

6. The method for computing morphology of cornea endothelium cells according to Claim 4 or 5, wherein in the step for, when an angle formed by successive two peripheral points and the center point is less than a specified angle, excluding from the peripheral points either of the two peripheral points, whichever longer in distance from the center point,
the specified angle is a value in a range of 20 to 40 degrees.

7. The method for computing morphology of cornea endothelium cells according to Claim 4, 5, or 6, further comprising, in the step for, when an angle formed by successive two peripheral points and the center point is less than a specified angle, excluding from the peripheral points either of the two peripheral points, whichever longer in distance from the center point,
a step for, when the angle formed by successive two peripheral points and the center point is greater than a value of 90 to 110 degrees, the center point is taken as a center point of a peripheral cornea endothelium cell. 8. The method for computing morphology of cornea endothelium cells according to any one of Claims 4 to 7, further comprising, in the step for, when a distance between the first point and the third point of successive three peripheral points is shorter than a distance between the center point and the second point, excluding the second point from the peripheral points;
a step for, when the angle formed by the first point, the center point, and the third point is greater than a value of 120 to 140 degrees, suppressing the second point from being excluded from the peripheral points. 9. The method for computing morphology of cornea endothelium cells according to any one of Claims 4 to 8, further comprising, in the step for determining a perpendicular line which proportionally divides a distance between the center point and a peripheral point by their average distances to the peripheral points, respectively for successive peripheral points, and determining intersection points of the resulting perpendicular lines as vertices of a profile of a cell,
a step for weighting proportional allotment with values of the square to fourth power of the average distances and effecting the proportional division with the values.
